# EUROPEAN PATENT SPECIFICATION

(11) **EP 2 705 810 B1**
(45) Date of publication and mention of the grant of the patent: **01.02.2017**
(21) Application number: 13183212.3
(22) Date of filing: 05.09.2013
(51) Int. Cl.: A61F 2/50, A61F 2/80, A61F 2/60

(54) **Method to make a negative cast for articular prostheses, corresponding apparatus and cast thus made**
Verfahren zur Herstellung eines negativen Abgusses für Gelenkprothesen und entsprechende Vorrichtung
Procédé de réalisation d'empreinte négative pour prothèses articulaires et appareil correspondant

(30) Priority: 05.09.2012 IT UD20120151
(43) Date of publication of application: 12.03.2014
(73) Proprietor: Marotto, Fulvio, 31100 Treviso (IT); Innovation Factory S.r.l., 34149 Trieste (IT); Ghedin, Patrizia, 31040 Volpago Del Montello (TV) (IT)
(72) Inventor: Marotto, Fulvio, 31100 Treviso (IT)
(74) Representative: Petraz, Gilberto Luigi

(56) References cited:
- WO-A2-2013/136284
- US-A- 3 520 002
- US-A- 5 503 543
- US-A1- 2004 076 700
- US-A1- 2010 115 757
- US-B1- 7 097 799

## Description

### FIELD OF THE INVENTION

The present invention concerns a method to make a cast from which to make an articular prosthesis, to be associated with the terminal segment or stump of an upper or lower amputated limb.

The present invention also concerns an apparatus to make said cast.

In particular, the present invention concerns a cast that bears the negative of the shape of the stump to which the articular prosthesis is to be associated.

### BACKGROUND OF THE INVENTION

It is known to make articular prostheses starting from a negative cast made of disposable material, such as plaster, of the terminal segment or stump of the upper or lower limb, with which they are to be associated.

In particular, in order to make articular prostheses, first of all the negative cast must be made, which is subsequently filled with a hardening material, for example plaster, or other similar materials, able to reproduce, positively, the shape of the stump in order to make a solid model thereof. The solid model is then used for the production of the articular prostheses, in particular to define the correct shape of the upper part, or socket, of the prostheses. In fact, the socket is shaped according to the solid model and, during use, surrounds and accommodates at least a terminal part of the stump.

Methods are known, which provide to make the negative cast by applying on the stump a moistened plastered gauze which is made to adhere to the stump and then made to solidify. In this way, the plastered gauze keeps the shape it assumes when it adheres to the stump and, after solidification, is able to reproduce the shape thereof in negative.

One disadvantage of this known method is that, because the shape of the stump is taken when the patient is resting, it is not possible to make a socket with a shape suitable to adhere correctly to the stump under load conditions, that is, in conditions of dynamic use, not only static or resting. For example, in the case of prostheses for lower limbs, the dynamic load conditions occur when the user is standing, or walking or running.

This creates discomfort for the user, because a loose socket, or one that does not have an optimum shape, makes the prosthesis uncomfortable and sometimes even painful. This discomfort limits the continuous use of the prosthesis, when walking.

In order to try to overcome this problem, it is known to use a sleeve or sock made of deformable material, such as silicon for example, interposed between the stump and the socket of the prosthesis. The sleeve or sock covers the stump and is intended to overcome the deformities and empty spaces that are created dynamically during use between the shape of the stump and the shape of the socket.

However, given its limited thickness, the sleeve or sock has the disadvantage that it does not overcome the discomfort of prolonged contact between the soft parts of the stump and the rigid part of the socket. Consequently, despite using the sleeve or sock, the user must necessarily and frequently suspend his/her activity in order to rest.

Document US-A-3,520,002 discloses an artificial limb having a rigid outer shell defining a cavity for receiving a limb stump. A stump socket is carried in the cavity and includes flexible pads positioned adjacent the inner wall of the outer shell of the artificial limb. An expansible foam material is inserted in the cavity in a liquid state, and is capable of expanding into a substantially rigid cellular supporting foam structure. An elastomeric member is positioned on the patient's limb stump in a stretched state for emphasizing scar tissue and bony areas of the limb. The limb stump is inserted within the socket prior to the expandible polymer expanding so that when such expands a rigid cellular supporting structure is produced having a cavity with an inner wall complementary in shape to the limb stump.

One purpose of the present invention is to perfect a method to make a cast for an articular prosthesis that allows to manufacture a prosthesis that can be used continuously by a user for relatively long periods of time, even a whole day.

Another purpose of the invention is to obtain an apparatus that allows to produce a cast for articular prostheses according to the method described above, to obtain a socket with a shape as similar as possible to that of the stump with which it has to enter into contact.

It is also a purpose of the present invention to obtain a cast for articular prostheses that allows to obtain a comfortable socket and that does not necessarily require to use a supplementary sleeve or sock, for example made of silicon.

### SUMMARY OF THE INVENTION

The present invention is set forth and characterized in the independent claims, while the dependent claims describe other characteristics of the invention or variants to the main inventive idea.

In accordance with the above purposes, a method according to the present invention is used for making a negative cast of a stump using deformable disposable material, used for the production of articular prostheses.

According to one feature of the present invention, the method comprises a step of compressing the deformable disposable material that makes the negative cast during its molding and solidification in conditions where the anatomy of the stump is reproduced in negative.

In this way, a pre-compressed negative cast is made, which reproduces the anatomical behavior in load conditions during dynamic use by the user.

The prosthesis that can be obtained from the pre-compressed negative cast according to the present invention is therefore extremely comfortable to wear, and the user can carry out prolonged activity on his/her feet, even for the whole day, wearing the prosthesis thus made, without fatigue or pain.

The present invention also concerns an apparatus to make a negative cast of a stump using deformable disposable material, used for the production of articular prostheses using a method according to the present invention.

The apparatus comprises molding means configured to cooperate with a deformable disposable material.

The molding means comprise a containing element able to contain the stump which, during use, is located at least partly in contact with the deformable disposable material which cooperates with the containing element to deform and reproduce the anatomical shape under load conditions of the stump when the deformable disposable material and the stump are subjected to a compressive force.

The present disclosure also concerns a negative cast of a stump, made pre-compressed using deformable disposable material for use in the production of articular prostheses.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a schematic view of a first form of embodiment of a method and apparatus according to the invention;
- fig. 2 is a schematic view of an embodiment of a method and apparatus to make a negative cast for articular prostheses, which is not part of the invention;
- fig. 3 is a variant of fig. 1.

### DETAILED DESCRIPTION OF SOME FORMS OF EMBODIMENT

With reference to the attached drawings, a method according to the present invention is used to make a negative cast 10 for articular prostheses. In this case, by way of non-restrictive example, a method to make a negative cast 10 of a stump 13 for prostheses for the lower limbs is described, using a deformable disposable material able to solidify and assume the anatomical shape of the limb, such as plaster for orthopedic use or similar or comparable materials.

The new and original technical solution, which obtains the above purposes and offers surprising and unforeseeable advantages, in technical terms, for the use and comfort of users, is based on the completely innovative idea, not deducible from the state of the art, of compressing the deformable disposable material that makes up the cast during its molding and solidification so that, once obtained, the negative cast is pre-compressed to the load conditions that are typical of dynamic use and is therefore comfortable, allowing prolonged activity.

In some forms of embodiment, the method comprises a step of preparing an apparatus 11 comprising molding means 12, configured to cooperate with at least a deformable disposable material 15, 20 able to reproduce in negative the three-dimensional shape of a stump 13 of a user. The negative cast 10 is obtained by means of the molding means 12, in a subsequent compression step, during which the stump 13 exerts a compressive force distributed substantially uniformly on the molding means 12 and on the deformable disposable material 15, 20, deforming them.

The deformation obtained during the compression step allows to obtain a negative cast 10 that reproduces the shape of the stump 13 under load, simulating a dynamic condition, for example as if the user were walking or running.

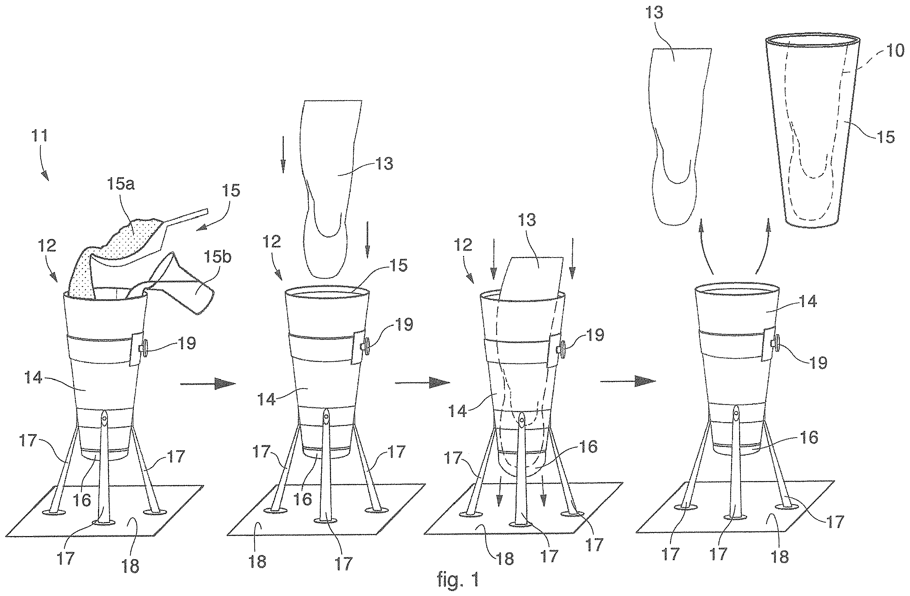

In a first form of embodiment of the invention, shown in fig. 1, the molding means 12 comprise a containing element or container 14, for example vase-shaped, and a deformable disposable material 15. The container 14, in this case, has an elongated shape, open at one end and closed at the opposite end by a bottom wall 16. Furthermore, the container 14 comprises support elements 17 that support it and keep it at a desired distance from a support plane 18. The distance must be at least sufficient to allow the bottom wall 16 of the container 14 to deform toward the support plane 18.

During the preparation of the molding means 12, the container 14 is rested on or attached to the support plane 18 and the deformable disposable material 15 is inserted inside it, in a quantity suitable to subsequently define the negative cast 10 of the stump 13. In this case, the deformable disposable material 15 is a fluid mixture of plaster 15a and water 15b that is poured inside the container 14 before the stump 13 is inserted.

The stump 13 is inserted after a certain waiting time, in the range of about 1.5 minutes, during which the mixture of plaster 15a and water 15b becomes more viscous and assumes the consistency of a plastic paste.

Other deformable disposable materials, having hardening properties, such as for example resins or polymer materials for orthopedic use, can be used instead of the mixture of plaster 15a and water 15b.

After the stump 13 has been inserted, during the compression step as described above, the patient loads all his/her weight on the stump 13, consequently exerting a compressive force on the mixture of plaster 15a and water 15b, comprised between the stump 13 and the walls of the container 14.

The compressive force entails a deformation of the mixture of plaster 15a and water 15b, which in turn exerts an equal and contrary compressive force distributed on the whole surface of the stump 13. The combination of the compressive forces allows the mixture of plaster 15a and water 15b to adhere perfectly to the stump 13. The loading of the user's weight on the stump 13 also causes the deformation of the bottom wall 16. This deformation is intended to allow a correct application of the load by the user who, forcing on a yielding surface, allows the stump 13 to assume exactly the shape that it normally has in dynamic conditions, such as for example when the user is walking or running. A rigid and non-yielding surface is not only uncomfortable and potentially painful, but would also modify the shape of the soft parts of the stump 13 which it comes into contact with, making it different from the shape normally assumed in said dynamic conditions.

The compression step exerted by the weight loaded by the patient ends when the mixture of plaster 15a and water 15b is completely solidified and therefore the negative cast 10 is made.

At the end of the compression step, the stump 13 and the negative cast 10 are removed from the container 14. The negative cast 10 is therefore ready to act in its turn as a container for making a solid model of the stump 13: afterward, the socket of the articular prosthesis that is to be obtained is shaped on the solid model.

The negative cast 10 thus obtained can be defined as pre-compressed and confers on the socket the correct shape so that it is able to distribute the weight resting on the stump 13 uniformly over its whole surface, in dynamic conditions of use.

In the form of embodiment shown in fig. 1, the molding means 12, in one form of embodiment, also comprise a lateral compression element 19, the function of which is to deform the container 14 laterally, narrowing it or widening it. This allows to modulate the pressure acting on the stump 13, respectively intensifying it or reducing it. A greater pressure on the stump 13 gives a greater definition of the negative cast 10.

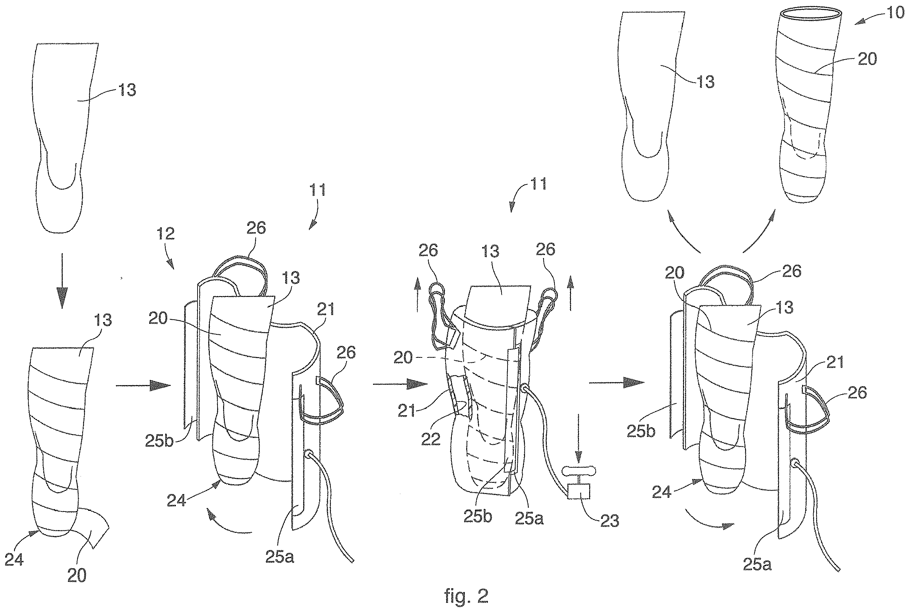

According to a variant, which is not part of the present invention, shown in fig. 2, the molding means 12 comprise an inflatable casing 21, which acts as a containing element for the stump 13, and a plastered gauze 20, or similar flexible material associated with disposable material such as plaster, which has the same function as the deformable disposable material 15.

The inflatable casing 21 in this case is made of flexible material and is provided with an internal chamber 22 to which to a filling device 23 is associated, for example a pump or a compressor. The filling device 23 is intended to make said inflatable casing 21 assume a first condition, not filled, in which it has a first dimensional bulk, and a second filled condition, in which it has a second dimensional bulk, bigger than the first bulk.

The step of preparing the molding means 12 provides that the plastered gauze 20 is soaked to obtain a subsequent hardening, for example in water, so that it is soft and flexible, and so that it can be wound around the stump 13, to cover at least some of the terminal part 24 thereof.

Once the stump 13 has been wound by the plastered gauze 20, it is inserted into the inflatable casing 21, when the latter is in its non-filled condition. In fact, in this condition the inflatable casing 21 is easily maneuverable and adjustable to surround the stump 13 covered by the plastered gauze 20. In the particular solution shown in fig. 2, the inflatable casing 21 is initially open longitudinally, that is, in the direction of development of the stump 13, and is closed around the latter to surround it. Two edges 25a and 25b, for example defining a tear-off closing, are overlapped to ensure the stable closing of the inflatable casing 21.

Other forms and types of inflatable casing 21 can be used, such as for example a casing defined by a closed and continuous wall that is fitted onto the stump 13 instead of wound and then closed.

After the stump 13 has been correctly positioned in the inflatable casing 21 the compression step takes place, which provides that the filling device 23 fills, even partly, the internal chamber 22 with a desired quantity of a fluid, in this case air, causing an increase in the bulk of the inflatable casing 21. The increase in bulk is blocked in a circumferential direction by the overlapping edges 25a and 25b and therefore develops radially, that is, in the direction of the stump 13, compressing it. The function of the compression is to deform the plastered gauze 20 to make it assume exactly the shape of the stump 13. It is therefore clear that the inflatable casing 21 can also perform the function of the lateral compression element 19 present in the variant shown in fig. 1.

A pair of handles 26, associated with the inflatable casing 21, allows to exert traction on the latter, and consequently another load on the whole stump 13.

In this way, during the compression step, before the plastered gauze 20 has solidified, the pressure and load borne by the stump 13 in dynamic conditions is correctly simulated.

The compression step ends when the plastered gauze 20 is completely solid.

At the end of the compression step, the inflatable casing 21 is returned to the unfilled condition, and both the stump 13 and the negative cast 10, consisting of the plastered gauze 20 which faithfully bears the shape of the stump 13, are removed from it.

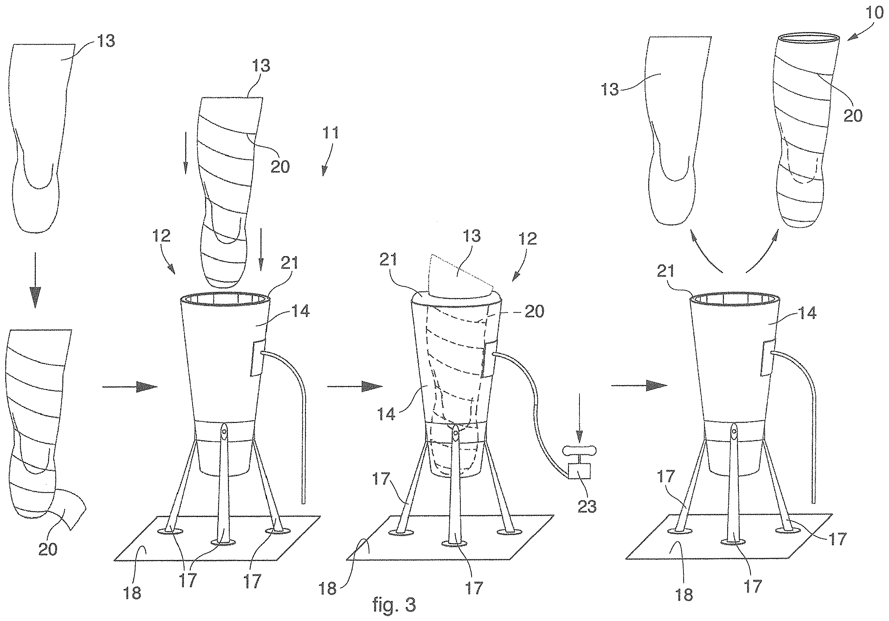

Another variant of the invention, shown in fig. 3, is substantially defined by a combination of the two variants described above. In this variant, the molding means 12 comprise the container 14, the wall of which integrates the inflatable casing 21, with which the filling device 23 is associated.

The method to make the negative cast 10 provides that the step of preparing the molding means 12 is similar to that provided for the variant in fig. 2, and that the negative cast 10 is made by compressing the plastered gauze 20.

The compression step provides that the user loads all his/her weight on the stump 13 inside the container 14, as in the variant in fig. 1, and that the lateral compression is performed by the inflatable casing 21, filled by the filling device 23.

It is clear that modifications and/or additions of parts may be made to the method and apparatus as described heretofore, without departing from the scope of the present invention.

It is also clear that, although the present invention has been described with reference to some specific examples, a person of skill in the art shall certainly be able to achieve many other equivalent forms of method and apparatus, having the characteristics as set forth in the claims and hence all coming within the field of protection defined thereby.

## Claims

1. Method to make a negative cast (10) of a stump (13) of a patient using deformable disposable material, said negative cast (10) being usable for the production of articular prostheses, said method comprising:
- providing molding means (12) comprising a containing element (14) open at one end and closed at the opposite end by a deformable bottom wall (16) and comprising a lateral compression element (19), said containing element (14) being configured to be laterally deformed by the lateral compression element (19), said containing element (14) containing at least a deformable disposable material (15);
- inserting the stump (13) in the containing element (14), immersing it into the deformable disposable material (15);
- a step of compressing the deformable disposable material (15) in which the patient loads all his/her weight on the stump (13) to make the negative cast (10) during molding and solidification of the deformable disposable material (15) in conditions where the anatomy of the stump (13) is reproduced in negative, wherein said deformable bottom wall (16) is deformed to allow the stump (13) to assume the shape that it normally has in dynamic conditions, further wherein the pressure acting on the stump (13) is modulated by laterally deforming the containing element (14) by the lateral compression element (19).

2. Method as in claim 1, which comprises, before the compression step, a step of preparing said molding means (12) in cooperation with said deformable disposable material (15), and in which said stump (13) is positioned inside said molding means (12), in order to make said deformable disposable material (15) adhere to said stump (13), wherein in the compression step, after said preparation step, a compressive force is applied to said deformable disposable material (15), comparable to the load to which said stump (13) is subjected in dynamic conditions, in order to deform said deformable disposable material (15), so that it reproduces in negative the anatomical shape of said loaded stump (13), in order to make said negative cast (10).

3. Method as in claim 2, wherein said compressive force is applied from inside said deformable disposable material (15) during the definition of the negative cast (10).

4. Method as in claim 3, wherein said compressive force is applied on the deformable disposable material (15) by loading the own weight of a user who immerses the stump (13) into the deformable disposable material and completely rests said stump (13) on the bottom wall (16) of said molding means (12).

5. Method as in claim 2, wherein said compressive force is applied from outside said deformable disposable material (15) during the definition of the negative cast (10).

6. Method as in claim 5, wherein said compressive force is applied on the deformable disposable material (15) as a consequence of a traction exerted on said molding means (12) in the direction of the stump (13).

7. Method as in any claim from 2 to 6, wherein said compressive force has a vertical component, acting on a terminal part (24) of said stump (13), and a horizontal component, acting laterally to the stump (13).

8. Apparatus to make a negative cast (10) of a stump (13) using disposable material according to a method as in any claims from 1 to 7, said negative cast (10) being usable for the production of articular prostheses, said apparatus comprising a deformable disposable material (15) and molding means (12) configured to cooperate with said deformable disposable material (15), wherein said molding means (12) comprise a containing element (14) open at one end and closed at the opposite end by a deformable bottom wall (16) and comprising a lateral compression element (19), said containing element (14) being configured to be laterally deformed by the lateral compression element (19), said containing element (14) being able to contain said deformable disposable material (15) and to receive said stump (13), which, during use, is immersed in said deformable disposable material (15) which cooperates with said containing element (14) in order to deform and reproduce the anatomical shape in load conditions of said stump (13) when said deformable disposable material (15, 20) and said stump (13) are subjected to a compressive force.

9. Apparatus as in claim 8, wherein said containing element is a container (14), supported by support elements (17) and said bottom wall (16) is able to be yieldingly deformed following the application of said compressive force.

10. Apparatus as in claim 9, wherein said deformable disposable material (15) is a mixture of plaster (15a) and water (15b), having hardening and solidifying properties, to be poured inside said container (14).

11. Apparatus as in claim 8, wherein said molding means (12) comprise an inflatable casing (21), having an internal chamber (22) and associated to a filling device (23) able to make said inflatable casing (21) assume a first condition, not filled, in which said internal chamber (22) is substantially empty, and a second condition, filled, in which said internal chamber (22) contains a desired quantity of a fluid, in order to increase the bulk of said inflatable casing (21) and to exert at least part of said compressive force on said stump (13) and on said deformable disposable material (20).

12. Apparatus as in claim 11, further comprising a pair of handles (26) associated to said inflatable casing (21) and adapted to exert a traction on said inflatable casing (21) and to apply at least part of said compressive force on said stump (13) and on said deformable disposable material (20).

13. Apparatus as in claim 8, 11 or 12, wherein said deformable disposable material comprises a plastered gauze (20) wrapped around at least a terminal part (24) of said stump (13).

14. Apparatus as in any of the claims from 8 to 13, wherein said containing element (14) is associated and/or integrated with said lateral compression element (19) able to exert at least part of said compressive force on said stump (13) and on said deformable disposable material (15, 20).

## Patentansprüche

1. Verfahren zur Herstellung eines negativen Abdrucks (10) eines Stumpfes (13) eines Patienten bzw. einer Patientin unter Verwendung von verformbarem Einwegmaterial, wobei der genannte negative Abdruck (10) für die Herstellung von Gelenkprothesen anwendbar ist, wobei das genannte Verfahren Folgendes umfasst:
- Bereitstellen von Formeinrichtungen (12) umfassend ein Behälterelement (14), das an einem Ende offen und am entgegengesetzten Ende durch eine verformbare Bodenwand (16) geschlossen ist und ein seitliches Druckelement (19) umfasst, wobei das genannte Behälterelement (14) dafür ausgelegt ist, um vom seitlichen Druckelement (19) seitlich verformt zu werden, wobei das genannte Behälterelement (14) zumindest ein verformbares Einwegmaterial (15) umfasst;
- Einführen des Stumpfes (13) in das Behälterelement (14), indem er in das verformbare Einwegmaterial (15) eingetaucht wird;
- einen Schritt des Zusammendrückens des verformbaren Einwegmaterials (15), in dem der Patient bzw. die Patientin mit seinem bzw. ihrem ganzen Gewicht den Stumpf (13) belastet, um den negativen Abdruck (10) während der Formung und der Erstarrung des verformbaren Einwegmaterials (15) unter Bedingungen zu erzeugen, in welchen die Anatomie des Stumpfes (13) in negativer Darstellung wiedergegeben wird, worin die genannte verformbare Bodenwand (16) verformt wird, um dem Stumpf (13) zu ermöglichen, die Form einzunehmen, die er üblicherweise unter dynamischen Bedingungen hat, ferner worin der Druck, der auf den Stumpf wirkt, moduliert wird, indem das Behälterelement (14) vom seitlichen Druckelement (19) seitlich verformt wird.

2. Verfahren nach Anspruch 1, das, vor dem Schritt des Zusammendrückens, einen Schritt des Vorbereitens der genannten Formeinrichtungen (12) in Zusammenarbeit mit dem genannten verformbaren Einwegmaterial (15) umfasst, und in welchem der genannte Stumpf (13) innerhalb der genannten Formeinrichtungen (12) positioniert ist, um das genannte verformbare Einwegmaterial (15) am genannten Stumpf (13) haften zu lassen, worin im Schritt des Zusammendrückens, nach dem genannten Schritt des Vorbereitens, eine Druckkraft auf das genannte verformbare Einwegmaterial (15) aufgebracht wird, die mit der Last vergleichbar ist, welcher der genannte Stumpf (13) unter dynamischen Bedingungen unterliegt, um das genannte verformbare Einwegmaterial (15) zu verformen, so dass es in negativer Darstellung die anatomische Form des genannten belasteten Stumpfes (13) wiedergibt, um den genannten negativen Abdruck (10) zu erzeugen.

3. Verfahren nach Anspruch 2, worin die genannte Druckkraft aus dem Inneren des genannten verformbaren Einwegmaterials (15) während der Bildung des negativen Abdrucks (10) aufgebracht wird.

4. Verfahren nach Anspruch 3, worin die genannte Druckkraft auf das genannte verformbare Einwegmaterial (15) aufgebracht wird, indem das Eigengewicht eines Benutzers geladen wird, der den Stumpf (13) in das genannte verformbare Einwegmaterial (15) eintaucht und den genannten Stumpf (13) auf die Bodenwand (16) der genannten Formeinrichtungen (12) vollständig stützt.

5. Verfahren nach Anspruch 2, worin die genannte Druckkraft aus dem Äußeren des genannten verformbaren Einwegmaterials (15) während der Bildung des negativen Abdrucks (10) aufgebracht wird.

6. Verfahren nach Anspruch 5, worin die genannte Druckkraft auf das genannte verformbare Einwegmaterial (15) als Folge eines Zugs aufgebracht wird, der auf die genannten Formeinrichtungen (12) in der Richtung des Stumpfes (13) ausgeübt wird.

7. Verfahren nach einem der Ansprüche 2 bis 6, worin die genannte Druckkraft eine vertikale Komponente, die auf einen Endteil (24) des genannten Stumpfes (13) wirkt, und eine horizontale Komponente, die seitlich auf den Stumpf (13) wirkt, aufweist.

8. Vorrichtung zur Herstellung eines negativen Abdrucks (10) eines Stumpfes (13) unter Verwendung von Einwegmaterial gemäß einem Verfahren nach einem der Ansprüche 1 bis 7, wobei der genannte negative Abdruck (10) für die Herstellung von Gelenkprothesen anwendbar ist, wobei die genannte Vorrichtung ein verformbares Einwegmaterial (15) und Formeinrichtungen (12) umfasst, die dafür ausgelegt sind, um mit dem genannten verformbaren Einwegmaterial (15) zusammenzuarbeiten, worin die genannten Formeinrichtungen (12) ein Behälterelement (14) umfassen, das an einem Ende offen und am entgegengesetzten Ende durch eine verformbare Bodenwand (16) geschlossen ist und ein seitliches Druckelement (19) umfasst, wobei das genannte Behälterelement (14) dafür ausgelegt ist, um vom seitlichen Druckelement (19) seitlich verformt zu werden, wobei das genannte Behälterelement (14) das genannte verformbare Einwegmaterial (15) enthalten und den genannten Stumpf (13) aufnehmen kann, der während der Benutzung im genannten verformbaren Einwegmaterial (15) eingetaucht ist, das mit dem genannten Behälterelement (14) zusammenarbeitet, um die anatomische Form unter Belastungsbedingungen des genannten Stumpfes (13) zu verformen und wiederzugeben, wenn das genannte verformbare Einwegmaterial (15, 20) und der genannte Stumpf (13) einer Druckkraft unterliegen.

9. Vorrichtung nach Anspruch 8, worin das genannte Behälterelement ein Behälter (14) ist, der von Stützelementen (17) abgestützt ist, und die genannte Bodenwand (16) nach der Anbringung der genannten Druckkraft nachgiebig verformt werden kann.

10. Vorrichtung nach Anspruch 9, worin das genannte verformbare Einwegmaterial (15) eine Mischung aus Gips (15a) und Wasser (15b) ist, die Härtungs- und Verfestigungseigenschaften aufweist und die in den genannten Behälter (14) gegossen werden kann.

11. Vorrichtung nach Anspruch 8, worin die genannten Formeinrichtungen (12) ein aufblasbares Gehäuse (21) umfassen, das eine innere Kammer (22) aufweist und mit einer Füllvorrichtung (23) verbunden ist, die dazu führen kann, dass das genannte aufblasbare Gehäuse (21) einen ersten, ungefüllten, Zustand, in dem die genannte innere Kammer (22) im Wesentlichen leer ist, und einen zweiten, gefüllten, Zustand einnimmt, in dem die genannte innere Kammer (22) eine gewünschte Menge einer Flüssigkeit enthält, um das Ausmaß des genannten aufblasbaren Gehäuses (21) zu erhöhen und zumindest einen Teil der genannten Druckkraft auf den genannten Stumpf (13) und auf das genannte verformbare Einwegmaterial (20) auszuüben.

12. Vorrichtung nach Anspruch 11, ferner umfassend ein Paar Griffe (26), die dem genannten aufblasbaren Gehäuse (21) zugeordnet sind und die dazu geeignet sind, einen Zug auf das genannte aufblasbare Gehäuse (21) auszuüben und zumindest einen Teil der genannten Druckkraft auf den genannten Stumpf (13) und auf das genannte verformbare Einwegmaterial (20) aufzubringen.

13. Vorrichtung nach Anspruch 8, 11 oder 12, worin das genannte verformbare Einwegmaterial eine Gipsgaze (20) umfasst, die um zumindest einen Endteil (24) des genannten Stumpfes (13) herum gewickelt ist.

14. Vorrichtung nach einem der Ansprüche 8 bis 13, worin das genannte Behälterelement (14) mit dem genannten seitlichen Druckelement (19) verbunden und/oder darin integriert ist, das zumindest einen Teil der genannten Druckkraft auf den genannten Stumpf (13) und auf das genannte verformbare Einwegmaterial (15, 20) ausüben kann.

## Revendications

1. Procédé de fabrication d'un contre-moule (10) d'un moignon (13) d'un patient utilisant un matériau déformable jetable, ledit contre-moule (10) étant utilisable pour la réalisation de prothèses articulaires, ledit procédé comprenant les étapes consistant à :
- se procurer des moyens de moulage (12) comprenant un élément formant conteneur (14) ouvert à une extrémité et fermé à l'extrémité opposée par une paroi inférieure déformable (16) et comprenant un élément de compression latérale (19), ledit élément formant conteneur (14) étant configuré pour être déformé latéralement par l'élément de compression latérale (19), ledit élément formant conteneur (14) contenant au moins un matériau déformable jetable (15) ;
- insérer le moignon (13) dans l'élément formant conteneur (14), l'introduire dans le matériau déformable jetable (15) ;
- et une étape de compression du matériau déformable jetable (15) au cours de laquelle le patient applique tous son poids sur le moignon (13) pour réaliser le contre-moule (10) durant le moulage et la solidification du matériau déformable jetable (15) dans des conditions telles que l'anatomie du moignon (13) soit reproduite en négatif, ladite paroi inférieure déformable (16) étant déformée pour permettre au moignon (13) d'adopter la forme qu'il a normalement dans des conditions dynamiques, la pression agissant en outre sur le moignon (13) étant modulée en déformant latéralement l'élément formant conteneur (14) à l'aide de l'élément de compression latérale (19).

2. Procédé selon la revendication 1, lequel comprend, avant l'étape de compression, une étape de préparation desdits moyens de moulage (12) en coopération avec ledit matériau déformable jetable (15) et dans lequel ledit moignon (13) est positionné à l'intérieur desdits moyens de moulage (12), afin de faire adhérer ledit matériau déformable jetable (15) audit moignon (13), dans lequel, durant l'étape de compression, après ladite étape de préparation, est appliquée sur ledit matériau déformable jetable (15) une force de compression qui est comparable à la charge à laquelle ledit moignon (13) est soumis dans des conditions dynamiques, afin de déformer ledit matériau déformable jetable (15), de manière à ce qu'il reproduise en négatif la forme anatomique dudit moignon (13) subissant la charge, afin de réaliser ledit contre-moule (10).

3. Procédé selon la revendication 2, dans lequel ladite force de compression est appliquée depuis l'intérieur dudit matériau déformable jetable (15) durant la création du contre-moule (10).

4. Procédé selon la revendication 3, dans lequel ladite force de compression est appliquée sur le matériau déformable jetable (15) en chargeant le propre poids d'un utilisateur qui introduit le moignon (13) dans le matériau déformable jetable et appuie complètement ledit moignon (13) sur la paroi inférieure (16) desdits moyens de moulage (12).

5. Procédé selon la revendication 2, dans lequel ladite force de compression est appliquée depuis l'extérieur dudit matériau déformable jetable (15) durant la création du contre-moule (10).

6. Procédé selon la revendication 5, dans lequel ladite force de compression est appliquée sur le matériau déformable jetable (15) suite à une traction exercée sur lesdits moyens de moulage (12) dans la direction du moignon (13).

7. Procédé selon l'une quelconque des revendications 2 à 6, dans lequel ladite force de compression a une composante verticale, agissant sur une partie terminale (24) dudit moignon (13), et une composante horizontale, agissant latéralement sur le moignon (13).

8. Appareil pour fabriquer un contre-moule (10) d'un moignon (13) en utilisant un matériau jetable suivant un procédé selon l'une quelconque des revendications 1 à 7, ledit contre-moule (10) étant utilisable pour la réalisation de prothèses articulaires, ledit appareil comprenant un matériau déformable jetable (15) et des moyens de moulage (12) configurés pour coopérer avec ledit matériau déformable jetable (15), dans lequel lesdits moyens de moulage (12) comprennent un élément formant conteneur (14) ouvert à une extrémité et fermé à l'extrémité opposée par une paroi inférieure déformable (16) et comprenant un élément de compression latérale (19), ledit élément formant conteneur (14) étant configuré pour être déformé latéralement par l'élément de compression latérale (19), ledit élément formant conteneur (14) étant apte à contenir ledit matériau déformable jetable (15) et à recevoir ledit moignon (13), lequel, durant l'utilisation, est introduit dans ledit matériau déformable jetable (15) lequel coopère avec ledit élément formant conteneur (14) afin de déformer et reproduire la forme anatomique dans des conditions de charge dudit moignon (13) quand ledit matériau déformable jetable (15, 20) et ledit moignon (13) sont soumis à une force de compression.

9. Appareil selon la revendication 8, dans lequel ledit élément formant conteneur est un conteneur (14) supporté par des éléments de support (17) et ladite paroi inférieure (16) est apte à être déformée par le poids suite à l'application de ladite force de compression.

10. Appareil selon la revendication 9, dans lequel ledit matériau déformable jetable (15) est un mélange de plâtre (15a) et d'eau (15b) ayant des propriétés de durcissement et de solidification, prévu pour être versé à l'intérieur dudit conteneur (14).

11. Appareil selon la revendication 8, dans lequel lesdits moyens de moulage (12) comprennent un boîtier gonflable (21) ayant une chambre interne (22) et associé à un dispositif de remplissage (23) apte à mettre ledit boîtier gonflable (21) dans un premier état, non rempli, dans lequel ladite chambre interne (22) est sensiblement vide, et un second état, rempli, dans lequel, ladite chambre interne (22) contient une quantité souhaitée d'un fluide, afin d'augmenter le volume dudit boîtier gonflable (21) et d'exercer au moins une partie de ladite force de compression sur ledit moignon (13) et sur ledit matériau déformable jetable (20).

12. Appareil selon la revendication 11, comprenant en outre une paire de poignées (26) associées audit boîtier gonflable (21) et aptes à exercer une traction sur ledit boîtier gonflable (21) et à appliquer au moins une partie de ladite force de compression sur ledit moignon (13) et sur ledit matériau déformable jetable (20).

13. Appareil selon la revendication 8, 11 ou 12, dans lequel ledit matériau déformable jetable comprend une gaze plâtrée (20) enroulée autour d'au moins une partie terminale (24) dudit moignon (13).

14. Appareil selon l'une quelconque des revendications 8 à 13, dans lequel ledit élément formant conteneur (14) est associé et/ou intégré avec ledit élément de compression latérale (19) apte à exercer au moins une partie de ladite force de compression sur ledit moignon (13) et sur ledit matériau déformable jetable (15, 20).
